(19) Europäisches Patentamt
European Patent Office
Office européen des brevets

(11) Veröffentlichungsnummer : **0 482 448 B1**

# (12) EUROPÄISCHE PATENTSCHRIFT

(45) Veröffentlichungstag der Patentschrift : **21.04.93 Patentblatt 93/16**

(51) Int. Cl.[5] : **C07D 417/04,** A23L 1/226

(21) Anmeldenummer : **91117354.0**

(22) Anmeldetag : **11.10.91**

## (54) Dimethylfuryl-dihydro-1,3,5-dithiazine, Verfahren zu ihrer Herstellung und ihre Verwendung.

(30) Priorität : **24.10.90 DE 4033809**

(43) Veröffentlichungstag der Anmeldung : **29.04.92 Patentblatt 92/18**

(45) Bekanntmachung des Hinweises auf die Patenterteilung : **21.04.93 Patentblatt 93/16**

(84) Benannte Vertragsstaaten : **AT CH DE ES FR GB IT LI NL SE**

(56) Entgegenhaltungen :
**EP-A- 0 186 026**
**US-A- 4 200 741**

(73) Patentinhaber : **HAARMANN & REIMER GMBH**
**Postfach 138**
**W-3450 Holzminden (DE)**

(72) Erfinder : **Brüning, Jürgen, Dr.**
**Sparenbergstrasse 39**
**W-3450 Holzminden (DE)**
Erfinder : **Emberger, Roland, Dr.**
**Bärenfang 4**
**W-3450 Holzminden (DE)**
Erfinder : **Güntert, Matthias, Dr.**
**Vogelsang 4**
**W-3450 Holzminden (DE)**
Erfinder : **Hopp, Rudolf, Dr.**
**Auf dem Gehrenkamp 28**
**W-3450 Holzminden (DE)**
Erfinder : **Köpsel, Manfred, Dr.**
**Schinkelstrasse 1**
**W-3450 Holzminden (DE)**
Erfinder : **Werkhoff, Peter, Dr.**
**Sieplerstrasse 24**
**W-3470 Höxter 1 (DE)**

(74) Vertreter : **Petrovicki, Wolfgang, Dr. et al**
**Bayer AG Konzernverwaltung RP Patente Konzern**
**W-5090 Leverkusen 1 Bayerwerk (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 482 448 B1

## Beschreibung

Die Erfindung betrifft neue Dimethylfuryl-dihydro-1,3,5-dithiazine, ein Verfahren zu ihrer Herstellung durch Cyclokondensation von 2-Furfural, Acetaldehyd, Ammoniak und Schwefelwasserstoff (oder Ammonsulfid) und die Verwendung dieser neuen Verbindungen als Aromastoffe. Insbesondere verleihen sie Nährungs- und Genußmitteln Kaffee- und Mokka-Aroma.

Die neuen Verbindungen entsprechen der Formel

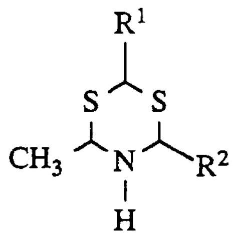

(I),

worin

$R^1$ und $R^2$ verschieden sind und für Methyl oder 2-Furyl stehen ( so daß also der eine Substituent Methyl und der andere 2-Furyl bedeutet).

Nachstehend bedeuten die Begriffe "Furfural" bzw. "Furyl" jeweils "2-Furfural" bzw. "2-Furyl".

Aus US-A-4200741 und EP-A-186026 sind bereits Dihydro-1,3,5-diathiazine als Aromastoffe bekannt, die allerdings keinen 2-Furyl substituenten tragen. Keine der gennanten Dokumente offenbaren jedoch ein Kaffee/Mokka-Aroma der dort offenbarten Verbindungen.

Die erfindungsgemäßen Verbindungen (I) können durch Umsetzung von Furfural, Acetaldehyd, Ammoniak und Schwefelwasserstoff gewonnen werden; Ammoniak und Schwefelwasserstoff können durch Ammonsulfid ersetzt werden. Da als unerwünschte Nebenprodukte das entsprechende Methyldifurylderivat und das entsprechende 2,4,6-Trimethylderivat, aber auch die analogen Tetrahydrothiadiazine entstehen können, wird man versuchen, die Reaktion durch Wahl der Molverhältnisse der Ausgangskomponenten in die gewünschte Richtung zu lenken. Das Molverhältnis Acetaldehyd/Furfural beträgt im allgemeinen 1,5 bis 5, vorzugsweise 2 bis 4, insbesondere 2,5 bis 3,5. Ammoniak setzt man in der Regel in geringem Überschuß, vorzugsweise in Mengen von > 1,0 bis 1,4, insbesondere 1,05 bis 1,15 Mol pro Mol Furfural, ein, wobei Ammoniak gasförmig eingeleitet oder, vorzugsweise, in Form einer wäßrigen Lösung (im allgemeinen 10 bis 33, vorzugsweise 20 bis 30 gew.-%ig) eingesetzt werden kann.

Schwefelwasserstoff wird man in einer etwa dem stöchiometrisch errechneten Verhältnis entsprechenden Menge einsetzen, wobei sich während des Einleitens die Sättigung des Reaktionsgemischs durch Austreten von Schwefelwasserstoff zu erkennen gibt. Die Reaktion läßt sich in Substanz, in Wasser oder in polaren (vorzugsweise wassermischbaren) inerten organischen Lösungsmitteln durchführen, wobei Wasser bevorzugt ist. Am einfachsten führt man die Reaktion in Wasser aus. Die Konzentration der Komponenten im Lösungsmittel ist nicht kritisch; jedoch wird man bestrebt sein, das Lösungsmittelvolumen aus praktischen Gründen in Grenzen zu halten: Pro Mol Furfural können im allgemeinen 10 bis 1.000, vorzugsweise 50 bis 200 ml Lösungsmittel als geeignete Menge betrachtet werden.

Aufgrund des Siedepunktes des Acetaldehyds von 20,2°C wird man für die Reaktion, sofern man nicht unter Druck arbeiten will, eine Temperatur zwischen 0 und 20, insbesondere von 5 bis 10°C wählen.

Die erfindungsgemäßen Verbindungen (I) lassen sich durch Extraktion mit einem organischen Lösungsmittel, z.B. Toluol, aus der wäßrigen Reaktionslösung, Abziehen des organischen Lösungsmittels und Reinigung mittels geeigneter Methode, beispielsweise Chromatographie, vorzugsweise Hochdruckflüssigkeitschromatographie (HPLC), isolieren. Da nebeneinander die beiden Isomeren (I) entstehen, hat man zu entscheiden, ob man für die geplante Anwendung das rohe Isomerengemisch als solches einsetzen oder - mit etwas höherem Trennungsaufwand - die reinen Isomeren isolieren will. (Arbeitet man nicht in wäßrigem Medium, ist die Methode auf geeignete Weise zu modifizieren).

Gegenstand der Erfindung sind also Dimethylfuryl-dihydro-1,3,5-dithiazine der Formel (I).

Weiterer Gegenstand der Erfindung ist ein Verfahren zur Herstellung dieser Verbindungen (I) durch Umsetzung von Furfural, Acetaldehyd, Ammoniak und Schwefelwasserstoff, gegebenenfals in Gegenwart eines Lösungsmittels.

Die erfindungsgemäßen Verbindungen (I) sind wertvolle Aromastoffe, die sich durch niedrige Geschmacksschwellenwerte auszeichnen. (Nachfolgend beziehen sich sämtliche Angaben in Prozenten, in ppm oder ppt auf das Gewicht).

So liegt der Geschmacksschwellenwert für 4,6-Dimethyl-2-furyl-dihydro-1,3,5-dithiazin, getestet von 5 speziell geschulten Prüfern, in 5 %iger wäßriger Zuckerlösung bei < 5 ppt, der Geschmacksschwellenwert für 2,6-Dimethyl-4-furyl-dihydro-1,3,5-dithiazin unter den gleichen Bedingungen bei < 100 ppt.

Die Geschmacksbeschreibung, bestimmt in 5 %iger wäßriger Zuckerlösung, für 4,6-Dimethyl-2-furyl-dihydro-1,3,5-dithiazin (bei einer Dosierung von 150 ppt): Mokka, brandig, Kaffee, Bitterschokolade, Kuchenkruste, stark gerösteter Kaffee.

Die Geschmacksbeschreibung, bestimmt wie oben beschrieben, für 2,6-Dimethyl-4-furyl-dihydro-1,3,5-dithiazin (bei einer Dosierung von 1 ppm): Kaffee, Mokka, Röstnote.

Die unter Verwendung der erfindungsgemäßen Verbindungen (I) hergestellten Aromakompositionen können im gesamten Nahrungsmittel- und Genußmittelbereich eingesetzt werden. Insbesondere sind sie geeignet für Getränke, Süß- und Backwaren.

Die erfindungsgemäßen Verbindungen (I) können in Mengen von 100 ppt bis 100 ppm, vorzugsweise 150 ppt bis 1 ppm, bezogen auf das verzehrfertige Nahrungsmittel, verwendet werden. Dabei können die erfindungsgemäßen Verbindungen (I) in reiner Form, der Einfachheit halber aber auch im Isomerengemisch, wie sie bei der Herstellung anfallen, eingesetzt werden.

Weiterer Gegenstand der Erfindung ist also die Verwendung der Verbindungen (I) als Aromastoffe.

In den nachfolgenden Beispielen beziehen sich Prozentangaben jeweils auf das Gewicht; Teile sind Gewichtsteile.

## Beispiele

### Herstellung

100 ml Wasser wurden vorgelegt, und unter Kühlung wurde bei einer Temperatur von 5 bis 10°C ein Gemisch aus 96 g (1 Mol) Furfural und 132 g (3 Mol) Acetaldehyd zudosiert. Anschließend wurden bei der gleichen Temperatur 75 g (1,1 Mol) wäßriger Ammoniak (25 %ig) zugegeben und bis zur Sättigung (etwa 7 Stunden) Schwefelwasserstoff eingeleitet. Das Gemisch blieb über Nacht stehen, wobei es zu einem Kristallbrei erstarrte. Die organischen Anteile wurden durch Zugabe von 200 ml Toluol in Lösung gebracht, und die organische Phase wurde von der wäßrigen Phase abgetrennt. Nach Abdestillieren des Lösungsmittels und der leichtflüchtigen Bestandteile - insbesondere 99 g (0,6 Mol) 2,4,6-Trimethyl-dihydro-1,3,5-dithiazin - blieb ein fester Rückstand von 34 g, aus dem durch Hochdruckflüssigkeitschromatographie (HPLC) ein Gemisch aus 4,6-Dimethyl-2-furyl-dihydro-1,3,5-dithiazin und 2,6-Dimethyl-4-furyl-dihydro-1,3,5-dithiazin abgetrennt wurde. Eine weitere HPLC-Trennung ergab die reinen Verbindungen im Verhältnis 95:5 des 2-Furyl- zum 4-Furylisomeren.

Für die HPLC-Trennungen wurde als stationäre Phase Kieselgel (100 Å, 10 µm), als mobile Phase Pentan verwendet. Die Säulenlänge betrug 25 cm, der Säulendurchmesser 2 cm.

Die erhaltenen Isomeren wurden NMR-spektroskopisch (400 MHz, $CDCl_3$, Tetramethylsilan als Standard) charakterisiert:

| 2-Furylisomer | 4-Furylisomer |
|---|---|
| δ = 7,379 ppm (1H, dd) | δ = 7,388 ppm (1H, dd) |
| = 6,35 ppm (2H, m) | = 6,35 ppm (2H, m) |
| = 5,552 ppm (1H, s) | = 5,304 ppm (1H, d) |
| = 4,272 ppm (2H, dq) | = 4,390 ppm (1H, q) |
| = 1,519 ppm (6H, d) | = 4,234 ppm (1H, dq) |
| = 0,9 ppm (1H, m) | = 1,560 ppm (3H, d) |
| | = 1,535 ppm (3H, d) |
| | = 1,2 ppm (1H, t) |

### Anwendung 1

Einem Instantkaffee (fertiges Getränk) wurden 6 ppb des nach obiger Vorschrift erhaltenen Gemisches

aus 4,6-Dimethyl-2-furyl- und 2,6-Dimethyl-4-furyl-dihydro-1,3,5-dithiazin zugesetzt. Die aromatisierte Probe hatte im Vergleich zur nicht aromatisierten Kontrollprobe ein deutlich typischeres und röstigeres Kaffeearoma.

Anwendung 2

Einem Malzkaffee (fertiges Getränk) wurden 10 ppb des in Anwendung 1 eingesetzten Gemisches zugesetzt. Die aromatisierte Probe hatte im Vergleich zur Kontrollprobe ein wesentlich typischeres und angenehmeres Kaffeearoma.

## Patentansprüche

1. Dimethylfuryl-dihydro-1,3,5-dithiazine der Formel

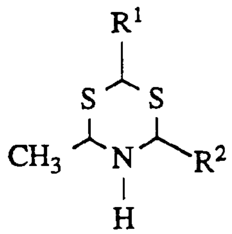

(I),

worin
$R^1$ und $R^2$ verschieden sind und für Methyl oder 2-Furyl stehen.

2. Verfahren zur Herstellung von Dimethylfuryl-dihydro-1,3,5-dithiazinen nach Anspruch 1 durch Umsetzung von Furfural, Acetaldehyd, Ammoniak und Schwefelwasserstoff bzw. Ammonsulfid, gegebenenfalls in Gegenwart eines Lösungsmittels.

3. Verfahren nach Anspruch 2, wonach das Molverhältnis Acetaldehyd/Furfural 1,5 bis 5 beträgt.

4. Verfahren nach Anspruch 2, wonach das Molverhältnis Acetaldehyd/Furfural 2 bis 4 beträgt.

5. Verfahren nach Anspruch 2, wonach das Molverhältnis Acetaldehyd/Furfural 2,5 bis 3,5 beträgt.

6. Verfahren nach Anspruch 2 bei einer Temperatur zwischen 0 und 20°C.

7. Verfahren nach Anspruch 1 in Wasser als Lösungsmittel.

8. Verwendung der Dimethylfuryl-dihydro-1,3,5-dithiazine nach Anspruch 1 als Aromastoffe.

## Claims

1. Dimethylfuryl-dihydro-1,3,5-dithiazines of the formula

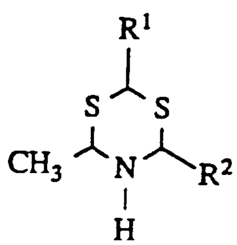

(I),

in which
$R^1$ and $R^2$ are different and represent methyl or 2-furyl.

2. Process for the preparation of dimethylfuryldihydro-1,3,5-dithiazines according to Claim 1 by reaction of furfural, acetaldehyde, ammonia and hydrogen sulphide or ammonium sulphide, if appropriate in the presence of a solvent.

3. Process according to Claim 2, according to which the molar ratio acetaldehyde/furfural is 1.5 to 5.

4. Process according to Claim 2, according to which the molar ratio acetaldehyde/furfural is 2 to 4.

5. Process according to Claim 2, according to which the molar ratio acetaldehyde/furfural is 2.5 to 3.5.

6. Process according to Claim 2 at a temperature between 0 and 20°C.

7. Process according to Claim 1 in water as a solvent.

8. Use of the dimethylfuryl-dihydro-1,3,5-dithiazines according to Claim 1 as flavourings.

**Revendications**

1. Diméthylfuryl-dihydro-1,3,5-dithiazines de formule

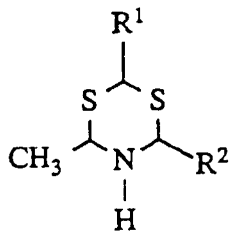

(I),

dans laquelle

$R^1$ et $R^2$ sont différents et représentent des groupes méthyle ou 2-furyle.

2. Procédé de production de diméthylfuryldihydro-1,3,5-dithiazines suivant la revendication 1, par réaction de furfural, d'acétaldéhyde, d'ammoniac et d'hydrogène sulfuré ou de sulfure d'ammonium, le cas échéant en présence d'un solvant.

3. Procédé suivant la revendication 2, dans lequel le rapport molaire acétaldéhyde/furfural a une valeur de 1,5 à 5.

4. Procédé suivant la revendication 2, dans lequel le rapport molaire acétaldéhyde/furfural a une valeur de 2 à 4.

5. Procédé suivant la revendication 2, dans lequel le rapport molaire acétaldéhyde/furfural a une valeur de 2,5 à 3,5.

6. Procédé suivant la revendication 2, mis en oeuvre à une température comprise entre 0 et 20°C.

7. Procédé suivant la revendication 1, mis en oeuvre dans l'eau utilisée comme solvant.

8. Utilisation des diméthylfuryl-dihydro-1,3,5-dithiazines suivant la revendication 1 comme arômes.